# EUROPEAN PATENT SPECIFICATION

(11) **EP 1 943 989 B1**
(45) Date of publication and mention of the grant of the patent: **29.04.2009**
(21) Application number: 07115301.9
(22) Date of filing: 30.08.2007
(51) Int. Cl.: A61F 9/007, A61B 17/34

(54) **Self-sealing cannula**
Selbstversiegelnde Kanüle
Canule auto-étanche

(30) Priority: 11.01.2007 US 622133
(43) Date of publication of application: 16.07.2008
(73) Proprietor: Alcon, Inc., 6221 Hünenberg (CH)
(72) Inventor: Attinger, Jurg, 8260 Stein am Rhein (CH)
(74) Representative: Hanna, Peter William Derek

(56) References cited:
- WO-A-96/23536
- US-A- 5 817 099
- US-A1- 2005 149 078
- US-A1- 2006 106 415
- US-A1- 2007 198 045

## Description

This invention relates to ophthalmic surgical equipment and more particularly to posterior segment ophthalmic surgical equipment.

### Background of the Invention

Microsurgical instruments typically are used by surgeons for any manipulations or removal of tissue from delicate and restricted spaces in the human body, particularly in surgery on the eye, and more particularly in procedures for manipulations or removal of the vitreous body, blood, scar tissue, or the crystalline lens. Such instruments may be hand-held, but often include a control console and a surgical handpiece with which the surgeon dissects, manipulates and/or removes the tissue. The handpiece has a surgical tool such as a vitreous cutter probe or an ultrasonic fragmentor for cutting or fragmenting the tissue and is connected to the control console by a long air pressure (pneumatic) line or power cable and by long conduits, cable, optical cable or flexible tubes for supplying an infusion fluid to the surgical site and for withdrawing or aspirating fluid and cut/fragmented tissue from the site. The cutting, infusion and aspiration functions of the handpiece are controlled by the remote control console that not only provides power for the surgical handpiece(s) (e.g., a reciprocating or rotating cutting blade or an ultrasonically vibrated needle), but also controls the flow of infusion fluid and provides a source of reduced pressure (relative to atmosphere) for the aspiration of fluid and cut/fragmented tissue. The functions of the console are controlled manually by the surgeon, usually by means of a foot-operated switch or proportional control.

During posterior segment surgery; the surgeon typically uses several instruments during the procedure. This requires that these instruments be inserted into, and removed out of the incision. This repeated removal and insertion can cause trauma to the eye at the incision site. To address this concern, cannulae were developed at least by the mid-1980s. These devices consist of a narrow tube with an attached hub. An incision is made, and the tube is inserted into the incision up to the hub, which acts as a stop, preventing the tube from entering the eye completely. Surgical instruments can be inserted into the eye through the tube, and the tube protects the incision from repeated contact by the instruments. In addition, the surgeon can use the instrument, by manipulating the instrument when the instrument is inserted into the eye through the tube, to help position the eye during surgery. Prior art cannulae have open tubes. As the interior of the eye is pressurized, vitreal fluids can flow out of the tube when the tube is not connected to an infusion device, or when an instrument is not inserted within the tube. Prior art cannulae sets include a removable plug that can be installed on the hub to prevent loss of vitreal fluids. Another prior art device includes a slit silicone diaphragm or cap on the outside of the hub. The slit provides an opening into the tube through which the surgical instrument can be inserted. While this diaphragm helps to prevent the loss of vitreal fluids out of the cannula, vitreous strands can get pulled back into the tube by the surgical instruments. When the cannula is removed from the incision, these strands get pulled into the incision.

Accordingly, a need continues to exist for a self-sealing cannula that does not require a separate plug and help prevent vitreal fluids from entering the cannula.

### Brief Summary of the Invention

The present invention improves upon prior art by providing a cannula having a tube and an enlarged rib or hub, in accordance with claims which follow. The hub is attached to the proximal end of the tube so that the distal end of the tube may be inserted into an incision up to the hub. The hub acts as a stop to prevent the entire cannula from entering the incision. The distal end of the tube contains a biased flap that seals the distal end of the tube while still allowing surgical instruments to be inserted into the eye through the tube.

Accordingly, an objective of the present invention is to provide an ophthalmic cannula.

Another objective of the present invention is to provide a self-sealing ophthalmic cannula.
A further objective of the present invention is to provide an ophthalmic cannula having a biased flap that seals the distal end of the cannula.

Other objectives, features and advantages of the present invention will become apparent with reference to the drawings, and the following description of the drawings and claims.

### Brief Description of the Drawings

FIG. 1 is an enlarged cross-section view of a prior art cannula.
FIG. 2 is an enlarged perspective view of the cannula of the present invention illustrating the valve in the closed position.
FIG. 3 is an enlarged perspective view of the cannula of the present invention illustrating the valve in the open position.

### Detailed Description of the Invention

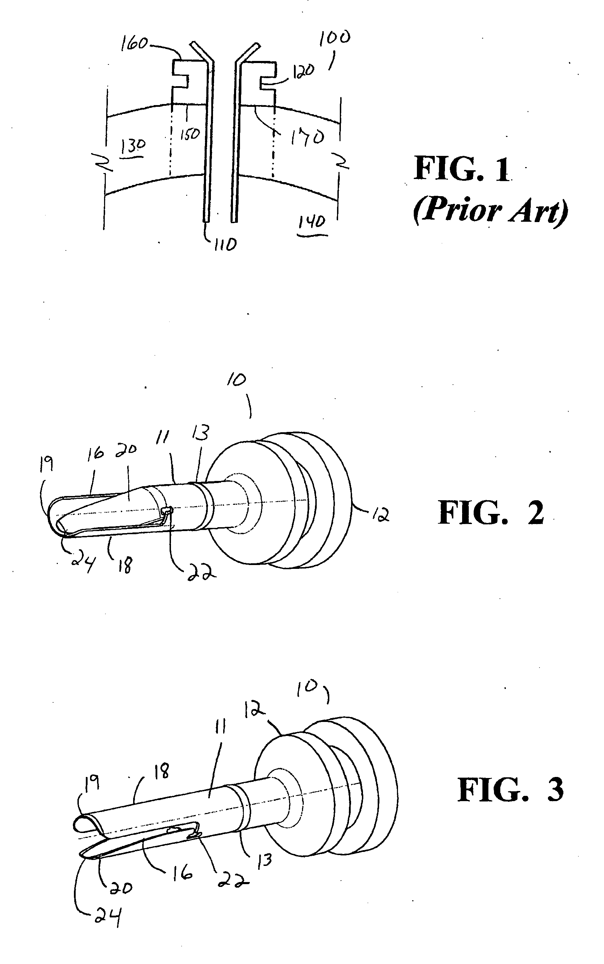

As best seen in FIG. 1, prior art cannula 100 consists of tube 110 and hub 120. Tube 110 is of sufficient length to extend through sclera 130 and enter posterior chamber 140. Hub 120 is generally cylindrical, with flat distal face 150 and proximal face 160.

As seen in FIGS. 2 and 3, cannula 10 of the present invention consists of tube 11 and enlarged hub 12. Tube 11 is of sufficient length to extend through sclera 130 and enter posterior chamber 140. Tube 11 and hub 12 are made from any suitable material such as metal or thermoplastic. The internal diameter of tube 11 may be of any suitable size, such as 20, 23 or 25 gauge or other. Tube 11 may contain rib 13 to help prevent cannula 10 from becoming dislodged from sclera 130. Tube 11 is longitudinally partially bisected by opposing slits 16 forming opposing sidewalls 18 and 20. Sidewall 20 is hingedly connected to the remainder of tube 11 by hinges 22 and sidewall 20 is formed so as to be biased about hinges 22 toward sidewall 1 when in a relaxed condition, as seen in FIG. 2. To assist in sidewall 20 nesting within sidewall 18, distal end 24 of sidewall 20 may be tapered. As seen in FIG. 3, sidewall 20 will pivot about hinges 22 and away from sidewall 18 in response to the insertion of an instrument (not shown) into tube 11 through the proximal end of tube 11 located at hub 12. Additionally, distal tip 19 of sidewall 18 may be sharpened to assist in the insertion of cannula 10 into sclera 130 without the use of a separate trocar or lance.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. A cannula (10), comprising a tube (11) having a distal end and a proximal end and a hub (12) connected to the tube at the proximal end, with means to prevent or reduce the flow of fluid out of the cannula in use, wherein said means comprises the distal end being partially longitudinally split to form a first sidewall (20) and a second sidewall (18), and a hinge (22) connecting the first sidewall to the remainder of the tube (11), the first sidewall being biased toward the second sidewall in a relaxed condition,
**characterized in that** the first sidewall (20) is tapered so as to nest within the second sidewall (18) in a relaxed condition.

2. The cannula of claim 1, wherein the distal tip (19) of the second sidewall (18) is sharpened to assist in insertion of the cannula in use.

## Patentansprüche

1. Kanüle (10), die ein Rohr (11) mit einem distalen Ende und einem proximalen Ende sowie eine Nabe (12) aufweist, die mit dem Rohr am proximalen Ende verbunden ist, mit einem Mittel zum Verhindern oder Verringern der Fluidströmung aus der im Einsatz befindlichen Kanüle, wobei dieses Mittel so ausgeführt ist, dass das distale Ende in Längsrichtung teilweise geteilt ist, um eine ersten Seitenwand (20) und eine zweite Seitenwand (18) zu bilden, und einem Scharnier (22), das die erste Seitenwand mit dem Rest des Rohrs (11) verbindet, wobei die erste Seitenwand im entspannten Zustand in Richtung der zweiten Seitenwand vorgespannt ist,
**dadurch gekennzeichnet, dass** die erste Seitenwand (20) konisch verjüngt ist, so dass sie im entspannten Zustand in die zweite Seitenwand (18) eingreift.

2. Kanüle nach Anspruch 1, bei der die distale Spitze (19) der zweiten Seitenwand (18) geschärft ist, um das Einführen der Kanüle bei der Anwendung zu unterstützen.

## Revendications

1. Canule (10) comportant un tube (11) ayant une extrémité distale et une extrémité proximale, et un moyeu (12) relié au tube au niveau de l'extrémité proximale, ayant des moyens pour empêcher ou réduire l'écoulement de fluide à l'extérieur de la canule en utilisation, dans laquelle lesdits moyens comprennent l'extrémité distale fendue partiellement longitudinalement pour former une première paroi latérale (20) et une seconde paroi latérale (18), et une articulation (22) reliant la première paroi latérale au reste du tube (11), la première paroi latérale étant rappelée vers la seconde paroi latérale dans un état relâché,
**caractérisée en ce que** la première paroi latérale (20) est effilée de manière à s'emboîter dans la seconde paroi latérale (18) dans un état relâché.

2. Canule selon la revendication 1, dans laquelle la pointe distale (19) de la seconde paroi latérale (18) est affutée pour aider à l'insertion de la canule en utilisation.
